# EUROPÄISCHE PATENTSCHRIFT

(11) **EP 4 319 928 B1**
(45) Veröffentlichungstag und Bekanntmachung des Hinweises auf die Patenterteilung: **03.06.2026**
(21) Anmeldenummer: 22723341.8
(22) Anmeldetag: 07.04.2022
(51) Int. Cl.: B06B 1/06, A61B 17/225, A61H 23/00, G10K 11/32, G10K 15/04

(54) **ELEKTROAKUSTISCHER WANDLER**
ELECTROACOUSTIC TRANSDUCER
TRANSDUCTEUR ÉLECTROACOUSTIQUE

(30) Priorität: 09.04.2021 DE 102021203544
(43) Veröffentlichungstag der Anmeldung: 14.02.2024
(73) Patentinhaber: Richard Wolf GmbH, 75438 Knittlingen (DE)
(72) Erfinder: BAUER, Edgar, 76703 Kraichtal (DE); KRAUSS, Werner, 75438 Knittlingen (DE); GAMER, Roland, 76676 Graben-Neudorf (DE)
(74) Vertreter: Patentanwälte Hemmer Lindfeld Frese Partnerschaft mbB
(86) Internationale Anmeldenummer: PCT/DE2022/200070
(87) Internationale Veröffentlichungsnummer: WO 2022/214150

(56) Entgegenhaltungen:
- CN-A- 109 365 253
- DE-B4- 102010 055 836
- DE-C1- 19 733 233
- DE-C1- 3 932 959
- US-A1- 2002 007 118

## Beschreibung

Die Erfindung betrifft einen elektroakustischen Wandler zum Erzeugen von Stoßwellen zur Behandlung des menschlichen oder tierischen Körpers.

Elektroakustische Wandler können nach unterschiedlichen physikalischen Prinzipien arbeiten. Die vorliegende Erfindung betrifft solche elektroakustischen Wandler zum Erzeugen von Stoßwellen zur Behandlung des menschlichen und tierischen Körpers, die zumindest unter Verwendung von piezoelektrischen Elementen aufgebaut sind. Derartige Wandler zählen in unterschiedlichen Bauformen zum Stand der Technik. Es gibt fokussierte, linienfokussierte, planare Wandler und Mischformen, die zum Stand der Technik zählen.

Aus DE 33 198 71 A1 ist ein piezoelektrischer Wandler zur Zerstörung von Konkrementen im Körperinneren bekannt, bei dem die piezoelektrischen Elemente auf einem Träger in Form einer Halbkugelschale angeordnet, elektrisch verbunden und mit Gießharz als Vergussmasse ausgefüllt sind. Durch die Anordnung der Elemente auf einer Kugelschale ist der Wandler selbstfokussierend. Die Wandlerinnenseite ist flüssigkeitsgefüllt und über eine Membran an den zu behandelnden Körper ankoppelbar. Das Gießharz muss die piezoelektrischen Elemente gegenüber der Flüssigkeit schützen, was problematisch ist.

Ein ähnlich aufgebauter elektroakustischer Wandler ist aus DE 195 43 741 C1 bekannt, bei diesem ist die Kunststoffvergussmasse durch eine Metallfolie als Feuchtigkeitsbarriere geschützt. Beiden Ausführungen gemeinsam ist, dass die piezoelektrischen Elemente in der Regel in mühsamer Handarbeit auf der Trägerkalotte befestigt und elektrisch mit einander verbunden werden müssen, wonach das Gießharz aufgefüllt wird, wozu ein spezielles Werkzeug erforderlich ist, welches den zur Ankoppelseite weisenden Teil der Gussform bildet. Dabei ist für jede Wandlerform und Größe ein gesondertes Werkzeug erforderlich, was aufwendig und teuer ist.

Aus DE 10 2010 055 836 B4 zählt es zum Stand der Technik, einen selbstfokussierenden elektroakustischen Wandler, der mit piezoelektrischen Elementen aufgebaut ist, mehrlagig aufzubauen. Dort sind die piezoelektrischen Elemente in Trägern angeordnet, die eine Vielzahl von Durchgangslöchern aufweisen, in denen jeweils ein piezoelektrisches Element gefasst und gehaltert ist. Auch bei dieser Bauart kann rückseitig ein Backing oder Stützkörper vorgesehen sein, um den mechanischen Halt des Wandlers zu gewährleisten, doch wird auch hier der Verbund durch eine Vergussmasse erzeugt, welche unter Verwendung eines speziellen an der Ankoppelseite anzuordnenden Werkzeugs eingefüllt wird. Das Werkzeug muss solange dort verbleiben, bis die Vergussmasse ausreichend ausgehärtet ist.
DE 197 33 233 C1 offenbart einen elektroakustischen Wandler mit einem kalottenförmigen Träger. Auf der Innenseite und der Außenseite des Trägers sind piezoelektrische Elemente angeordnet. Zwischenräume der piezoelektrischen Elemente sind mit einem Vergussmaterial ausgefüllt.
DE 39 32 959 C1 offenbart einen piezoelektrischen Wandler zur Erzeugung fokussierter Ultraschall-Stoßwellen. Auf einem Träger des Wandlers ist eine Vielzahl von piezoelektrischen Wandlerelementen angeordnet. Ein zwischen dem Träger und einem Gehäuse des Wandlers gebildeter Raum, in dem die piezoelektrischen Elemente angeordnet sind, ist mit einem hochisolierenden Medium gefüllt.
CN 109 365 253 A1 offenbart einen piezoelektrischen Wandler zum Enteisen. Der Wandler umfasst eine Rückwand und eine Dichtung, zwischen denen zwei piezoelektrische Elemente angeordnet sind. Jeweils zwischen der Rückwand und dem benachbarten piezoelektrischen Element, den beiden piezoelektrischen Elementen sowie der Dichtung und dem benachbarten piezoelektrischen Element sind eine Elektrode angeordnet.
US 2007/007118 A1 offenbart einen Ultraschallwandler mit einem piezoelektrischen Empfangsresonator und einem Senderesonator. Die beiden Resonatoren sind in einem gemeinsamen Gehäuse angeordnet. In Schallrichtung hinter den Resonatoren ist eine Dämpfungsschicht angeordnet. Zwischen den beiden Resonatoren ist eine akustische Anpassungsschicht angeordnet.

Vor diesem Hintergrund liegt der anmeldungsgemäßen Erfindung die Aufgabe zu Grunde, einen elektroakustischen Wandlerzum Erzeugen von Stoßwellen zur Behandlung des menschlichen oder tierischen Körpers der vorerwähnten Bauart, das heißt der unter Verwendung piezoelektrischer Elemente aufgebaut ist, so auszugestalten, dass die Herstellung vereinfacht ist. Darüber hinaus soll ein Verfahren zum Herstellen eines solchen elektroakustischen Wandlers bereitgestellt werden.

Der vorrichtungsmäßige Teil dieser Aufgabe wird durch einen elektroakustischen Wandler mit den in Anspruch 1 angegebenen Merkmalen gelöst. Ein Verfahren zur Herstellung eines solchen Wandlers ist durch die in Anspruch 13 angegebenen Merkmale gekennzeichnet. Vorteilhafte Ausgestaltungen ergeben sich aus den Unteransprüchen, der nachfolgenden Beschreibung und den Zeichnungen.

Der erfindungsgemäße elektroakustische Wandler zum Erzeugen von Stoßwellen zur Behandlung des menschlichen oder tierischen Körpers ist mit piezoelektrischen Elementen ausgestattet, die in mindestens einem Träger innerhalb eines Gehäuses angeordnet sind. Der zwischen den piezoelektrischen Elementen und dem Gehäuse gebildete Freiraum ist mit einer Vergussmasse ausgefüllt.

Grundgedanke der vorliegenden Erfindung ist es, durch ein Gehäuse, welches Teil des späteren elektroakustischen Wandlers bildet, das sonst aufwendige Gießwerkzeug zu ersetzen. Die erfindungsgemäße Lösung ermöglicht es somit sowohl in Kleinserie als auch einzeln kostengünstig elektroakustische Wandler der eingangs genannten Art herzustellen, welche praktisch ohne Werkzeug hergestellt werden können, bei denen jedoch die piezoelektrischen Elemente dicht und fest durch eine aushärtende Vergussmasse fixiert und gehalten sind. Die Vergussmasse kann aber auch aus Gel bestehen, ein Hochspannungsöl sein oder ein in Flüssigkeit schwebend gehaltenes sandähnliches Pulver. Dabei ergibt sich ein Verbund für zwischen den piezoelektrischen Elementen, dem oder den Trägern und dem Gehäuse, welcher ein stabiles Ganzes bildet. Ein solches Gehäuse, welches beispielsweise im 3D-Druck hergestellt werden kann, ermöglicht es, den Wandler in seiner äußeren Form und in seinem inneren Aufbau quasi beliebig zu variieren, ohne hierzu aufwendige Werkzeugänderungen erforderlich zu machen. Gemäß der Erfindung ersetzt somit das Gehäuse das Gießwerkzeug und hat darüber hinaus den Vorteil, dass es nach Aushärten der Vergussmasse noch nicht einmal entfernt werden muss. Es hat weiter den Vorteil, dass auch Vergussmassen verwendet werden können, welche nicht aushärten, welche flüssig oder pastös sind. Sofern das Gehäuse, wie bei einer Vielzahl von Ausführungen, keine tragende Funktion hat, kann dies kostengünstig und gegebenenfalls auch in Einzelfertigung hergestellt werden.

Bevorzugt wird gemäß einer Weiterbildung der Erfindung der Freiraum mit einem aushärtenden Kunststoff ausgegossen. Ein solcher aushärtender Kunststoff kann beispielsweise ein Thermoplast sein, der nach Abkühlen innerhalb des Gehäuses aushärtet oder ein Duroplast, welcher chemisch aushärtet, ähnlich den aus dem Stand der Technik bekannten Vergussmassen. Ein solcher Kunststoff kann vorteilhaft mit festigkeitserhöhenden Fasern, z. B. Glasfasern, Kohlefasern, Partikeln oder anderen makroskopischen Körpern vernetzt sein.

Vorteilhaft wird das Gehäuse gemäß einer Weiterbildung der Erfindung dabei so ausgebildet, dass es mindestens eine Öffnung zum Einfüllen der Vergussmasse und mindestens eine weitere Öffnung für den Gasaustritt beim Einfüllen der Vergussmasse aufweist. Es versteht sich, dass je nach Bauform auch mehrere Einfüll- bzw. Entgasungsöffnungen im Gehäuse vorgesehen sein können. Dabei kann die Öffnung für den Gasaustritt durch Evakuierung in diesem Bereich den Füllvorgang unterstützen. Bei einem aushärtenden Kunststoff können diese Öffnungen nach dem Verfüllen des Freiraums mit der Vergussmasse und dem Aushärten offen bleiben, handelt es sich jedoch um Vergussmassen, welche in flüssiger, pastöser oder einer Mischform verbleiben, so können diese Öffnungen verschließbar vorgesehen sein, sei es, dass ein entsprechendes Füllventil in der Einfüllöffnung und ein Stopfen für die Austrittsöffnung vorgesehen ist oder aber dass diese Öffnungen zum Abschluss der Fertigung gezielt verschlossen werden, sei es durch Verschweißen, Verkleben oder dergleichen.

Vorteilhaft ist das Gehäuse mehrteilig aufgebaut, und besteht aus mindestens zwei Gehäuseteilen, welche nach Eingliederung von dem Träger bzw. den Trägern mit den piezoelektrischen Elementen fest miteinander verbunden werden. Dies erfolgt vorzugsweise stoffschlüssig, sei es durch Kleben, Schweißen oder dergleichen.

Gemäß der Erfindung ist das Gehäuse als geschlossenes Gehäuse ausgebildet und ersetzt das Gießwerkzeug vollständig. Die Vergussmasse wird sicher vom Gehäuse aufgenommen. Gemäß einer alternativen Ausführungsform die nicht Teil der beanspruchten Erfindung ist, kann vorgesehen sein, dass das Gehäuse teiloffen ausgebildet ist, das heißt, nicht vollständig geschlossen ist. In diesem Fall ist die offene Seite auf der von der Ankoppelseite gegenüberliegend abgewandten Seite vorgesehen, also auf der Rückseite des Gehäuses. Hier kann je nach Konstruktionsausführung des Wandlers auf eine Gehäusewandung verzichtet werden, wenn beispielsweise ein Planarwandler mit aushärtendem Kunststoff auszugießen ist, da dann in diesem Bereich kein Gießwerkzeug erforderlich ist. Unter teiloffenem Gehäuse im Sinne der vorliegenden Erfindung ist also nur ein solches Gehäuse zu verstehen, welches an einer Seite offen ist, auf der beim Ausfüllen des Frei-raums mit der Vergussmasse kein Werkzeug erforderlich ist, wie dies typischerweise an der Rückseite bei speziellen Bauformen der Fall sein kann.

Es kann günstig sein, sowohl das Gehäuse als auch den oder die Träger aus Kunststoff auszubilden, diese können im 3D-Druck her-gestellt oder auch als Spitzgussteile ausgebildet sein, wenn größere Stückzahlen herzustellen sind. Insbesondere Teile des Gehäuses können auch vorteilhaft aus Metall bestehen, aber auch der oder die Träger, wobei dann in der Regel besondere Vorkehrungen hinsichtlich der elektrischen Isolierungen zu treffen sind. Vorteilhaft aus Metall besteht die Ankoppelseite, insbesondere wenn hier eine flüssigkeitsgefüllte Vorlaufstrecke anschließt, um zu verhindern, dass Feuchtigkeit durch das Gehäuse in die Vergussmasse diffundiert. Um die Festigkeit der Bauteile zu erhöhen kann es vorteilhaft sein, hier Verbundwerkstoffe einzusetzen, insbesondere auch im Bereich des oder der Träger, welche wesentliche Teile des späteren Verbundes bilden. Auch bei Ausgestaltungen, bei welchen die Vergussmasse nicht aushärtend ist, bildet der Träger den wesentlichen mechanischen Verbund zwischen den Piezoelementen. Der oder die Träger sind dann zweckmäßigerweise form-, kraft- und/oder stoffschlüssig am Gehäuse festgelegt.

Insbesondere bei der Verwendung von Kunststoff können Gehäuseteile vorteilhaft durch Tiefziehen gebildet werden. Dieses Verfahren eignet sich für Serien kleiner und mittlerer Größen. Um bei Verwendung eines Kunststoffgehäuses sicherzustellen, dass keine Flüssigkeit von der Ankoppelstrecke in den Wandler diffundiert, kann gemäß einer vorteilhaften Weiterbildung der Erfindung das Gehäuse zumindest an der Ankoppelseite mit einer flüssigkeitsdichten Metallschicht versehen sein. Eine solche Metallschicht kann in Form einer Folie oder auch aufgedampft vorgesehen sein und ist vorzugsweise innenseitig des Gehäuses angeordnet, da dann keine besonderen Vorkehrungen zum Schutz der Metallschicht vorzusehen sind. Andererseits kann die Metallschicht auch an der Ankoppelseite vorgesehen sein, dann wird in der Regel aber zumindest ein schützender Lack oder eine weitere darüberliegende Schutzschicht vorzusehen sein.

Insbesondere bei Wandlern kleinerer Baugröße ist es vorteilhaft, das Gehäuse an der Ankoppelseite mit einem Gelpolster als Koppelstrecke zu versehen. Vorteilhaft ist ein solches Gelpolster auswechselbar, um unterschiedliche Eindringtiefen insbesondere bei selbstfokussierenden Wandlern zu ermöglichen.

Gemäß einer Weiterbildung der Erfindung kann das Gehäuse so ausgebildet sein, dass eine vorzugsweise durch die Vorder- und Rückseite verlaufende Durchbrechung gebildet ist, die zur Aufnahme einer Kamera, eines Ultraschallwandlers oder eines Sensors vorgesehen ist. Je nach Anforderungen können eine oder mehrere solche Durchbrechungen vorgesehen sein. Dabei wird eine Durchbrechung vorteilhaft vollständig von dem Gehäuse abgeschlossen, so dass trotz der Durchbrechung das Innere des Gehäuses durch die Gehäusewandung geschützt bleibt.

Die erfindungsgemäße Gehäuseausbildung ermöglich es, individuell angepasste Gehäuse auch in kleinsten Stückzahlen kostengünstig zu fertigen. Insoweit kann das Gehäuse des erfindungsgemäßen Wandlers vorteilhaft anwendungsspezifisch anatomisch angepasst ausgebildet sein, und zwar zumindest an der Ankoppelseite aber nicht notwendigerweise nur an dieser. Wenn sowohl Gehäuse als auch Träger im 3D-Druck hergestellt werden, dann kann die Gehäuseform und die Anordnung der piezoelektrischen Elemente darin praktisch beliebig konfiguriert werden. So ist es denkbar, zur Behandlung der Extremitäten das Gehäuse wannenförmig auszubilden und die Ankoppelseite durch den Boden der Wanne zu bilden. Auch können die piezoelektrischen Elemente innerhalb des Gehäuses so angeordnet werden, dass eine teilfokussierte Wellenfront entsteht, wie sie beispielsweise zur Weichteilbehandlung zweckmäßig ist.

Zur Anordnung der piezoelektrischen Elemente innerhalb des Trägers ist es vorteilhaft, wenn der Träger die Piezoelemente zumindest abschnittsweise umfänglich umfasst, so dass die piezoelektrischen Elemente an ihrer Vorderseite und ihrer Rückseite frei und somit zugänglich für die elektrische Verdrahtung sind. Typischerweise werden piezoelektrische Elemente derselben Trägerebene parallelgeschaltet, wobei die Anschlüsse hintereinanderliegender Trägerebenen gesondert herausgeführt sind, um die zeitliche Abfolge der Spannungsbeaufschlagung und damit die Ausbreitung der Wellenfronten steuern zu können.

Die piezoelektrischen Elemente können je nach Auslegung des Schallwandlers einzeln, in Gruppen oder zusammen ansteuerbar sein. Entsprechend hat die elektrische Verdrahtung zu erfolgen. Eine große Varianz der Ansteuerung kann durch Einzelansteuerung erzielt werden, was jedoch eine entsprechend aufwändige Verdrahtung erfordert. Bei Einzel- oder Gruppenansteuerung ist die Ansteuerung in zeitlicher Varianz möglich, wodurch praktisch beliebige Wellenfronten erzeugt werden können.

Der erfindungsgemäße elektroakustische Wandler wird typischerweise so hergestellt, dass zunächst die piezoelektrischen Elemente in einem oder mehreren Trägern angeordnet werden. Ein Träger ist ein Bauteil, welches eine Vielzahl von Ausnehmungen aufweist, in denen jeweils ein piezoelektrisches Element klemmbefestigbar ist. Es erfolgt dann zweckmäßigerweise die elektrische Verdrahtung der Elemente wonach das so entstandene Gebilde in das typischerweise mehrteilige Gehäuse eingegliedert wird, welches dann durch Kleben oder Ultraschallschweißen fest und dicht verschlossen wird, indem die Gehäuseteile miteinander verbunden werden. Danach wird dann die Vergussmasse eingefüllt, die typischerweise nach einer vorbestimmten Zeit aushärtet, wonach der elektroakustische Wandler fertiggestellt ist.

Es sei darauf hingewiesen, dass neben dem vorbeschriebenen mit Ausnehmungen versehenen Träger auch zusätzliche Stützkörper innerhalb des Gehäuses vorgesehen sein können. Auch kann das Gehäuse selbst einen solchen Stützkörper bilden, es ist dann entsprechend stabil ausgebildet.

Durch den erfindungsgemäßen Aufbau des elektroakustischen Wandlers können praktisch beliebige Formen und Strukturen hergestellt werden. So können mehrfach gekrümmte Wandler, segmentierte Linienwandler, Wandler mit stufenförmig abgesetztem Gehäuse sowie Wandler mit integrierten Einzelkalotten und andere Spezialbauformen kostengünstig hergestellt werden, insbesondere wenn die Träger und das Gehäuse im 3D-Druckverfahren hergestellt sind. Auch können die Wandler in ihrer Gesamtheit mit vorzugsweise außerhalb des Gehäuses angeordneten akustischen Linsen versehen sein oder die piezoelektrischen Elemente vorzugsweise innerhalb des Gehäuses mit entsprechenden Linsen versehen sein.

Die Erfindung ist nachfolgend anhand von in den Figuren dargestellten Ausführungsbeispielen näher erläutert. Es zeigen:
- Fig. 1: in stark vereinfachter Schnittdarstellung einen selbstfokussierenden Wandler gemäß der Erfindung,
- Fig. 2: einen aus zwei hintereinander angeordneten Trägern mit piezoelektrischen Elementen aufgebauten linienförmig fokussierenden Wandler in Darstellung gemäß Figur 1und,
- Fig. 3: in stark vereinfachter Schnittdarstellung einen anatomisch angepassten Wandler für den Fuß und,
- Fig. 4a - 4d: in stakt vereinfachter schematischer Darstellung diverse Wandlerformen.

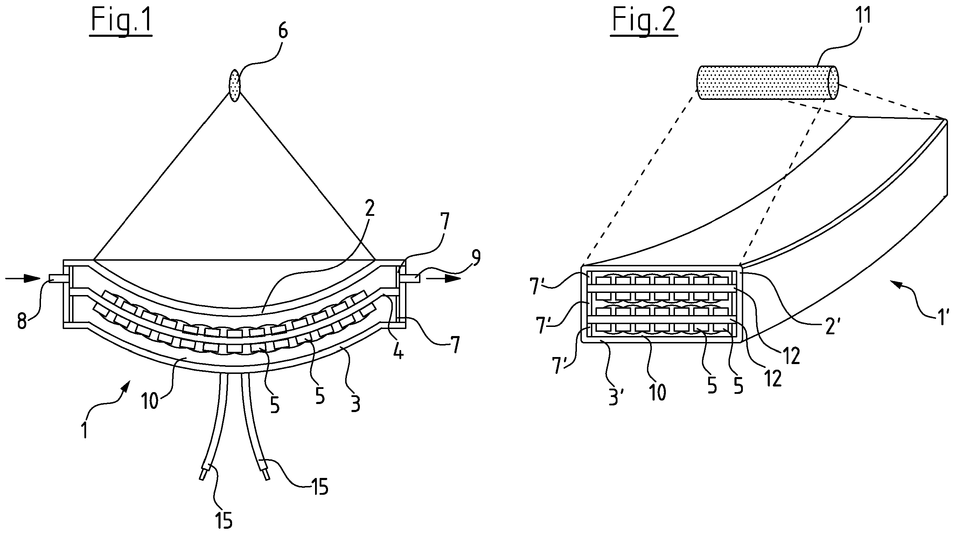

Bei dem in Figur 1 dargestellten elektroakustischen Wandler 1 handelt es sich um einen selbstfokussierenden Wandler, der ein aus Kunststoff bestehendes Gehäuse 2, 3 aufweist, welches zweiteilig ausgebildet ist und aus einem ersten, die Ankoppelseite des Wandlers 1 bildenden Gehäuseteil 2 besteht, welches topfförmig ausgebildet ist, einen zur Ankoppelseite gerichtete konkave Form aufweist und im Übrigen zylindrisch ist. Abgeschlossen wird der zylindrische Teil des Gehäuses durch einen rückseitigen Gehäuseteil 3, der ebenfalls aus Kunststoff besteht und durch Ultraschallschweißen fest und dicht in dem Gehäuseteil 2 verbunden ist. Innerhalb des Gehäuses 2, 3 ist ein Träger 4 angeordnet, in dem eine Vielzahl von piezoelektrischen Elementen 5 angeordnet sind. Die piezoelektrischen Elemente 5 haben im Wesentlichen eine zylindrische Form, sie sind umfangseitig in Ausnehmungen des Trägers 4 form- und kraftschlüssig festgelegt und an ihren Stirnseiten elektrisch verdrahtet. Sie sind so innerhalb des kalottenförmigen Trägers 4 angeordnet, dass bei gleichzeitiger elektrischer Beaufschlagung der Elemente 5 eine akustische Druckwelle erzeugt wird, deren Fokus 6 mit Abstand zur Ankoppelseite gegenüber dem konkaven Gehäuseteil 2 liegt.

Der Träger 4 mit den darin angeordneten piezoelektrischen Elementen 5 ist formschlüssig innerhalb des Gehäuses 2, 3 festgelegt, und zwar durch zwei zylindrische Ringe 7, welche mit ihrer Außenseite an der Innenseite des Gehäuses 2, 3 anliegen und mit einer Stirnseite an der vorderen Innenseite des Gehäuseteils 2 bzw. an der hinteren Innenseite des Gehäuseteils 3 anliegen. Zwischen den anderen Stirnseiten der Ringe 7 ist der Träger 4 formschlüssig eingegliedert. In dem vorderen Gehäuseteil 2 sind im zylindrischen Bereich umfänglich Anschlüsse 8, 9 vorgesehen, wobei der Anschluss 8 eine Öffnung zum Einfüllen einer Vergussmasse in das Gehäuse 2, 3 vorgesehen ist und der Anschluss 9 zum Anschluss einer Vakuumleitung bzw. als Ausgang für die beim Einfüllen der Vergussmasse in das Gehäuse 2, 3 austretende Luft. Über den Anschluss 8 wird also die Vergussmasse in das Gehäuse 2, 3 eingefüllt, der Einfüllvorgang kann durch Anschluss einer Vakuumpumpe an den Anschluss 9 unterstützt werden, zumindest erfolgt über den Anschluss 9 die Entgasung, das heißt das durch die eingefüllte Vergussmasse 10 verdrängte Gasvolumen entweicht durch diesen Anschluss 9.

Die Vergussmasse 10 wird unter Druck durch den Anschluss 8 in das Gehäuse 2, 3 eingefüllt, nachdem der Träger 4 mit den darin angeordneten piezoelektrischen Elementen 5 zwischen den Ringen 7 innerhalb des Gehäuses 2, 3 fixiert und die Gehäuseteile 2, 3 fest und dicht durch Kleben oder Schweißen miteinander verbunden sind. Dabei sind die elektrischen Anschlüsse der piezoelektrischen Elemente 5 bereits verdrahtet, die entsprechenden Anschlussleitungen 15 sind rückseitig dicht aus dem Gehäuse 2, 3 herausgeführt. Nachdem die Vergussmasse 10 ausgehärtet ist, sind die Anschlüsse 8, 9 durch die darin befindliche harte Vergussmasse 10 verschlossen. Im Übrigen ist durch die Vergussmasse ein fester zusammenhängender Körper gebildet, der nun in ein entsprechendes medizinisches Gerät eingebaut werden kann, wobei die konkave Außenseite des Gehäuseteils 2 die Ankoppelseite bildet, an der ein entsprechend geformter Gelkörper (nicht dargestellt) lösbar befestigt ist. Alternativ kann hier eine flüssigkeitsgefüllte Ankoppelstrecke mit Membran vorgesehen sein, dann ist der Gehäuseteil 2 vorzugsweise an der Innenseite mit einer diffusionsdichten Metallschicht versehen, um ein Eindringen von Flüssigkeit in den Wandler 1 zu vermeiden.

Bei dem anhand von Figur 2 dargestellten elektroakustischen Wandler 1' besteht das Wandlergehäuse 2', 3' ebenfalls aus zwei Gehäuseteilen, nämlich einen vorderen, zur Ankoppelseite gerichteten Gehäuseteil 2' und einem rückseitigen Gehäuseteil 3'. Das in Figur 2 dargestellte Gehäuse 2'/3' hat die Form eines zylindrischen Ringabschnittes und ist für einen linienfokussierenden Wandler 1' vorgesehen. Innerhalb des Gehäuses sind zwei Träger 12 angeordnet, die jeweils eine Vielzahl von piezoelektrischen Elementen 5 aufweisen, die jeweils trägerseitig parallel verdrahtet, jedoch getrennt rückseitig über nicht dargestellten Anschlussleitungen herausgeführt sind. Auch bei dieser Ausführung sind die Träger 12 durch in Draufsicht im Wesentlichen rechteckige Ringe 7' formschlüssig innerhalb des Gehäuses 2', 3' angeordnet. Die Anordnung ist dabei so, dass die piezoelektrischen Elemente 5 die erzeugte Schallwelle linienförmig fokussieren, der Fokusbereich ist in Figur 2 mit 11 gekennzeichnet. Im Übrigen ist der anhand von Figur 2 dargestellte Wandler 1' in gleicher Weise wie der gemäß Figur 1 aufgebaut. Die beiden Träger 12 mit darin eingegliederten piezoelektrischen Elementen 5 sind durch eine Vergussmasse 10 fest miteinander und mit dem umgebenden Gehäuse 2', 3' verbunden. Dieses Gehäuse 2', 3' ersetzt somit beim Gießvorgang das Gießwerkzeug. Die Gehäuseteile 2', 3' selbst sowie auch die Träger können kostengünstig z.B. im 3D-Druck gefertigt werden, so dass auch individuelle Gehäuseformen in Einzelfertigung wirtschaftlich zu realisieren sind.

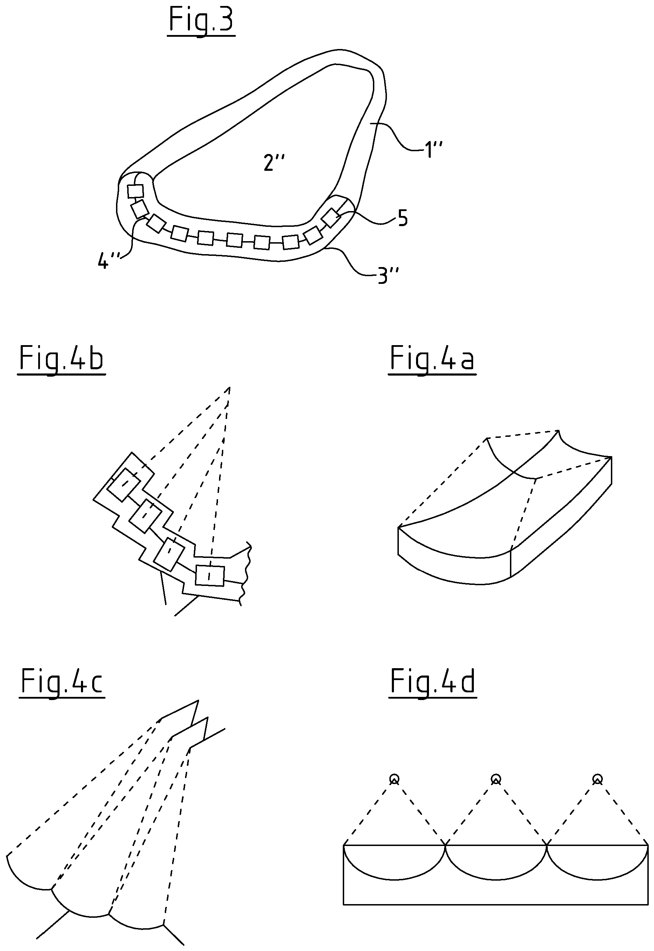

Anhand von Figur 3 ist schematisch dargestellt, wie ein anatomisch angepasster Wandler 1" ausgebildet sein kann. Der dort dargestellte Wandler 1" ist an die untere Form eines Fußes angepasst. In den Randbereichen, in welchen der Wandler 1" gewölbt ausgebildet ist um die Seite des Fußes umfassen zu können, sind die darin befindlichen piezoelektrischen Elemente 5 mit akustischen Zerstreuungslinsen (nicht dargestellt) versehen. Hierdurch soll eine ungewünschte Fokussierung in diesem Bereich verhindert werden. In analoger Weise kann durch Anordnen von Sammellinsen der umgekehrte Effekt erzielt werden. Diese akustischen Linsen sind zweckmäßigerweise gehäuseseitig vorgesehen und können an der Innenseite des oberen Gehäuseteils 2" aber auch an der Außenseite oder beiden Seiten vorgesehen sein. Die piezoelektrischen Elemente 5 sind auch bei dieser Ausführungsvariante in einen Träger 4" eingegliedert, welcher innerhalb des durch die Gehäuseteile 2" und 3" gebildeten Gehäuses formschlüssig gehalten ist. Der freie Raum zwischen den piezoelektrischen Elementen 5 und dem Gehäuse 2", 3" ist vollständig mit aushärtendem Kunststoff ausgefüllt.

Anhand der Figuren 4a bis 4d sind beispielhaft weitere elektroakustische Wandler dargestellt, um zu verdeutlichen, welche Vielfalt von Gehäuseformen mit dem vorliegenden Aufbau realisierbar ist. In Figur 4a ist ein zweiachsig gekrümmtes Gehäuse dargestellt, in dem die piezoelektrischen Elemente der Gehäuseform folgend einen Fokus erzeugen, welcher einer gekrümmten Linie entspricht.

In Figur 4b ist der Wandler stufenförmig angeordnet, um eine Aufspreizung der einzelnen piezoelektrischen Elemente in der Tiefe und damit eine Defokussierung zu erreichen.

Bei der anhand von Figur 4c dargestellten Anordnung sind mehrere linienförmig fokussierende Wandler in einem gemeinsamen Gehäuse angeordnet.

Die Ausführung gemäß Figur 4d zeigt einen Wandler mit mehreren nebeneinander liegenden punktförmig fokussierenden Abschnitten.

Die vorstehend beschriebenen elektroakustischen Wandler sind ausschließlich für medizinische Anwendungen ausgelegt und dienen zum Erzeugen von Stoßwellen, wie sie zum Behandeln des menschlichen und tierischen Körpers in der Medizin Anwendung finden. Zur Ankopplung des Wandlers an den Körper ist typischerweise eine flüssigkeits- oder gelgefüllte Vorlaufstrecke, zum Beispiel in Form eines Gelkissens, vorgesehen, wie dies zum Stand der Technik zählt.

### Bezugszeichenliste

- 1, 1', 1"-: Elektroakustischer Wandler
- 2, 2' -: Vorderer Gehäuseteil
- 3, 3' -: Hinterer Gehäuseteil
- 4, 4" -: Träger
- 5 -: Piezoelektrische Elemente
- 6 -: Fokus
- 7, 7' -: Ringe
- 8 -: Eingangsanschluss
- 9 -: Ausgangsanschluss
- 10 -: Vergussmasse
- 11 -: Fokusbereich
- 12 -: Träger in Fig. 2
- 15 -: Anschlussleitung

## Patentansprüche

1. Elektroakustischer Wandler (1, 1') zum Erzeugen von Stoßwellen zur Behandlung des menschlichen oder tierischen Körpers, mit piezoelektrischen Elementen (5), die in mindestens einem Träger (4, 12) innerhalb eines Gehäuses (2, 3; 2', 3') angeordnet sind, wobei ein zwischen den piezoelektrischen Elementen (5) und dem Gehäuse (2, 3; 2', 3') gebildeter Freiraum mit einer Vergussmasse (10) ausgefüllt ist, **dadurch gekennzeichnet, dass** das Gehäuse (2, 3; 2', 3') als geschlossenes Gehäuse ausgebildet ist und ein Gießwerkzeug für die Vergussmasse ersetzt.

2. Elektroakustischer Wandler nach Anspruch 1, bei dem der Freiraum mit aushärtendem Kunststoff (10) ausgegossen ist.

3. Elektroakustischer Wandler nach Anspruch 1 oder 2, bei dem das Gehäuse (2, 3; 2', 3') mindestens eine Öffnung (8) zum Einfüllen der Vergussmasse (10) und mindestens eine weitere Öffnung (9) für den Gasaustritt beim Einfüllen der Vergussmasse (10) aufweist.

4. Elektroakustischer Wandler nach einem der vorhergehenden Ansprüche, bei dem das Gehäuse (2, 3; 2', 3') aus mindestens zwei Gehäuseteilen (2, 3; 2', 3') gebildet ist, die vorzugsweise stoffschlüssig miteinander verbunden sind.

5. Elektroakustischer Wandler nach einem der vorhergehenden Ansprüche, bei dem das Gehäuse (2, 3; 2', 3') und/oder der Träger (4; 12) aus Kunststoff, Metall oder einem Verbundwerkstoff gebildet sind.

6. Elektroakustischer Wandler nach einem der vorhergehenden Ansprüche, bei dem mindestens ein Gehäuseteil (2, 3; 2', 3') durch Tiefziehen, Spritzguss- oder im 3D-Druck-Verfahren gebildet ist.

7. Elektroakustischer Wandler nach einem der vorhergehenden Ansprüche, bei dem das Gehäuse (2, 3; 2', 3') zumindest an der Ankoppelseite vorzugsweise innenseitig mit einer flüssigkeitsdichten Metallschicht versehen ist.

8. Elektroakustischer Wandler nach einem der vorhergehenden Ansprüche, bei dem das Gehäuse (2, 3; 2', 3') auf der Ankoppelseite mit einem vorzugsweise auswechselbaren Gelpolster als Koppelstrecke versehen ist.

9. Elektroakustischer Wandler nach einem der vorhergehenden Ansprüche, bei dem das Gehäuse (2, 3; 2', 3') mindestens eine vorzugsweise durch die Vorder- und Rückseite verlaufende Durchbrechung zur Aufnahme einer Kamera, eines Ultraschallwandlers, oder eines Sensors aufweist.

10. Elektroakustischer Wandler nach einem der vorhergehenden Ansprüche, bei dem das Gehäuse (2, 3; 2', 3') zumindest an der Ankoppelseite anwendungsspezifisch anatomisch angepasst ausgebildet ist.

11. Elektroakustischer Wandler nach einem der vorhergehenden Ansprüche, bei dem der Träger (4; 12) die piezoelektrischen Elemente (5) zumindest abschnittsweise umfänglich umfasst und die piezoelektrischen Elemente (5) an ihrer Vorderseite und an Ihrer Rückseite elektrisch angeschlossen sind.

12. Elektroakustischer Wandler nach einem der vorhergehenden Ansprüche, bei dem die in dem Träger (4,12) angeordneten piezoelektrischen Elemente (5) einzeln, in Gruppen oder zusammen elektrisch ansteuerbar sind.

13. Verfahren zum Herstellen eines Wandlers (1; 1')nach einem der vorhergehenden Ansprüche, bei dem piezoelektrische Elemente (5) in mindestens einem Träger (4; 12) angeordnet und elektrisch angeschlossen werden, bei dem der mindestens eine mit piezoelektrischen Elementen (5) bestückte Träger (4; 12) in einem geschlossenen Gehäuse (2, 3; 2', 3') angeordnet wird, und bei dem der zwischen den piezoelektrischen Elementen (5), dem Träger (4; 12) und dem Gehäuse (2, 3; 2', 3') gebildete Freiraum mit einer vorzugsweise aushärtenden Vergussmasse (10) gefüllt wird, wobei das Gehäuse ein Gießwerkzeug für die Vergussmasse ersetzt.

## Claims

1. An electroacoustic transducer (1, 1') for the generation of shock waves for the treatment of the human or animal body, the electroacoustic transducer comprising piezoelectric elements (5), which are arranged in at least one carrier (4, 12) within a housing (2, 3; 2', 3'), wherein a free space formed between the piezoelectric elements (5) and the housing (2, 3; 2', 3') is in-filled with a casting compound (10), **characterised in that** the housing (2, 3; 2', 3') is formed as a closed housing and replaces a casting tool for the casting compound.

2. The electroacoustic transducer according to claim 1, in which the free space is in-filled with hardening plastic (10).

3. The electroacoustic transducer according to claim 1 or 2, in which the housing (2, 3; 2', 3') has at least one opening (8) for the in-filling of the casting compound (10), and at least one further opening (9) for the escape of gas when in-filling the casting compound (10).

4. The electroacoustic transducer according to one of the preceding claims, in which the housing (2, 3; 2', 3') is formed from at least two housing parts (2, 3; 2', 3'), which are preferably connected to one another in an integrally bonded manner.

5. The electroacoustic transducer according to any one of the preceding claims, in which the housing (2, 3; 2', 3') and/or the carrier (4; 12) is formed from plastic, metal or a composite material.

6. The electroacoustic transducer according to any one of the preceding claims, in which at least one housing part (2, 3; 2', 3') is formed by deep drawing, injection moulding, or with the 3D printing method.

7. The electroacoustic transducer according to any one of the preceding claims, in which the housing (2, 3; 2', 3'), at least on the coupling side, is provided preferably on an inner side with a liquid-tight metal layer.

8. The electroacoustic transducer according to any one of the preceding claims, in which the housing (2, 3; 2', 3') is provided on the coupling side with a preferably exchangeable gel pad as a coupling section.

9. The electroacoustic transducer according to any one of the preceding claims, in which the housing (2, 3; 2', 3') has at least one aperture, preferably running through the front and rear side, for receiving a camera, an ultrasound transducer, or a sensor.

10. The electroacoustic transducer according to any one of the preceding claims, in which the housing (2, 3; 2', 3'), at least on the coupling side, is configured so as to be anatomically adapted to a specific application.

11. The electroacoustic transducer according to any one of the preceding claims, in which the carrier (4; 12) circumferentially surrounds the piezoelectric elements (5), at least in some sections, and the piezoelectric elements (5) are electrically connected on their front side and on their rear side.

12. The electroacoustic transducer according to any one of the preceding claims, in which the piezoelectric elements (5) arranged in the carrier (4, 12) are electrically actuatable individually, in groups, or all together.

13. A method for producing a transducer (1; 1') according to any one of the preceding claims, in which the piezoelectric elements (5) are arranged and electrically connected in at least one carrier (4; 12), in which the at least one carrier (4; 12), populated with piezoelectric elements (5), is arranged in a closed housing (2, 3; 2', 3'), and in which the free space formed between the piezoelectric elements (5), the carrier (4; 12) and the housing (2, 3; 2', 3') is in-filled with a preferably hardening casting compound (10), wherein the housing replaces a casting tool for the casting compound.

## Revendications

1. Convertisseur électroacoustique (1, 1') pour la génération d'ondes de choc destinées au traitement du corps humain ou animal, avec des éléments piézoélectriques (5) disposés dans au moins un support (4, 12) à l'intérieur d'un boîtier (2, 3 ; 2', 3'), un espace libre formé entre les éléments piézoélectriques (5) et le boîtier (2, 3 ; 2', 3') étant rempli d'une masse de moulage (10), **caractérisé en ce que** le boîtier (2, 3 ; 2', 3') est conçu comme un boîtier fermé et remplace un outil de coulée pour la masse de moulage.

2. Convertisseur électroacoustique selon la revendication 1, dans lequel du plastique durcissable (10) est coulé dans l'espace libre.

3. Convertisseur électroacoustique selon la revendication 1 ou 2, dans lequel le boîtier (2, 3 ; 2', 3') comporte au moins une ouverture (8) pour y verser la masse de moulage (10) et au moins une autre ouverture (9) pour la sortie de gaz lors de l'intégration de la masse de moulage (10).

4. Convertisseur électroacoustique selon l'une des revendications précédentes, dans lequel le boîtier (2, 3 ; 2', 3') est constitué d'au moins deux éléments de boîtier (2, 3 ; 2', 3'), de préférence reliés par liaison de matière.

5. Convertisseur électroacoustique selon l'une des revendications précédentes, dans lequel le boîtier (2, 3 ; 2', 3') et/ou le support (4 ; 12) sont en plastique, en métal ou en composite.

6. Convertisseur électroacoustique selon l'une des revendications précédentes, dans lequel au moins une partie du boîtier (2, 3 ; 2', 3') est formée par emboutissage, moulage par injection ou impression 3D.

7. Convertisseur électroacoustique selon l'une des revendications précédentes, dans lequel le boîtier (2, 3 ; 2', 3') est revêtu de préférence, au moins sur le côté accouplement, d'une couche métallique étanche au liquide.

8. Convertisseur électroacoustique selon l'une des revendications précédentes, dans lequel le boîtier (2, 3 ; 2', 3'), côté accouplement, est muni d'un coussin en gel de préférence remplaçable faisant office de ligne de couplage.

9. Convertisseur électroacoustique selon l'une des revendications précédentes, dans lequel le boîtier (2, 3 ; 2', 3') présente au moins une perforation passant de préférence par les côtés avant et arrière et destinée à recevoir une caméra, un transducteur à ultrasons ou un capteur.

10. Convertisseur électroacoustique selon l'une des revendications précédentes, dans lequel le boîtier (2, 3 ; 2', 3') est formé de manière à être anatomiquement adaptable au moins du côté accouplement.

11. Convertisseur électroacoustique selon l'une des revendications précédentes, dans lequel le support (4 ; 12) comprend au moins partiellement les éléments piézoélectriques (5) et les éléments piézoélectriques (5) sont raccordés électriquement par l'avant et l'arrière.

12. Convertisseur électroacoustique selon l'une des revendications précédentes, dans lequel les éléments piézoélectriques (5) disposés dans le support (4, 12) peuvent être commandés électriquement, individuellement, en groupes ou ensemble.

13. Procédé de fabrication d'un convertisseur (1 ; 1') selon l'une des revendications précédentes, dans lequel les éléments piézoélectriques (5) sont disposés et raccordés électriquement dans au moins un support (4 ; 12), au moins un support (5) équipé d'éléments piézoélectriques (4 ; 12) étant placé dans un boîtier fermé (2, 3 ; 2', 3'), et l'espace libre formé entre les éléments piézoélectriques (5), le support (4 ; 12) et le boîtier (2, 3 ; 2', 3') est rempli d'une masse de moulage de préférence durcissable (10), le boîtier remplaçant un outil de coulée pour la masse de moulage.
